(19) Europäisches Patentamt
European Patent Office
Office européen des brevets

(11) Veröffentlichungsnummer: **0 191 096**
**B1**

(12) # EUROPÄISCHE PATENTSCHRIFT

(45) Veröffentlichungstag der Patentschrift: **13.09.89**

(51) Int. Cl.⁴: **C 07 F 15/00**, A 61 K 31/28

(21) Anmeldenummer: **85904433.1**

(22) Anmeldetag: **24.07.85**

(86) Internationale Anmeldenummer: **PCT/EP 85/00369**

(87) Internationale Veröffentlichungsnummer: **WO 86/00905 (13.02.86 Gazette 86/04)**

(54) **TUMORHEMMEND WIRKENDE RUTHENIUMVERBINDUNGEN.**

(30) Priorität: **24.07.84 CH 3594/84**
**04.07.85 CH 2907/85**

(43) Veröffentlichungstag der Anmeldung: **20.08.86 Patentblatt 86/34**

(45) Bekanntmachung des Hinweises auf die Patenterteilung: **13.09.89 Patentblatt 89/37**

(84) Benannte Vertragsstaaten: **AT BE CH DE FR GB IT LI LU NL SE**

(56) Entgegenhaltungen:
**GB-A-956 242**

**Collection of Czechoslovak Chemical Communications, volume 26, no. 5, 1961, Prague (CS). F. Kralik et al.: "Komplexverbindungen des Rutheniums mit fünfzähligen heterocyclischen Basen II. Reacktionen des Ruthenium (III)-Chlorids mit Pyrazol und Imidazol", pages 1298-1304, see page 1299**
**Chemical Abstracts, volume 93, no. 4, 28 July 1980, Columbus, Ohio (US). J. Dehand et al.: "Chloronitrile complexes of ruthenium (III) and ruthenium (IV) and their reactivity towards several ligands", see page 640, abstract 36122a & Inorg. Chim. Acta 1979, 37(2) 249-53**
**American Chemical Society Symposium Series, volume 140, 1980. M.J. Clarke: "The Potential of Ruthenium in Anticancer Pharmaceuticals", pages 157-180, see page 174, paragraph 2 to page 178, paragraph 1**

(73) Patentinhaber: **ASTA Pharma AG, Weismüllerstrasse 45, D-6000 Frankfurt am Main 1 (DE)**

(72) Erfinder: **KELLER, Heimo, J., Angelweg 28, D-6900 Heidelberg (DE)**
Erfinder: **KEPPLER, Bernhard, Richard- Wagner-Strasse 9a, D-6830 Schwetzingen (DE)**

(56) Entgegenhaltungen: (Fortsetzung)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

EP 0 191 096 B1

## Beschreibung

Die Erfindung bezieht sich auf Arzneimittelzubereitungen enthaltend tumorhemmend wirkende Rutheniumverbindungen und Verfahren zur Behandlung von Krebskrankheiten.

## Stand der Technik

Von den Verbindungen der Platinmetalle hat sich die Komplexverbindung cis-Diammindichloroplatin(II) als potentes Antitumormittel bewährt. In tierexperimentellen Studien wird auch von Verbindungen der anderen Platinmetalle eine Hemmung des Tumorwachstums beobachtet (z. B. T. Giraldi et al., Cancer Res. 37 [1977] 2662 - 6). Von L. J. Anghileri (Z. Krebsforsch. 83 [1975] 213 - 7) wird über eine tumorhemmende Wirkung von Ruthenium-Rot berichtet. Bei H. J. Clarke, ACS Symp. Ser. 140 [1980] 157 - 180 wird die potentielle Eignung von Rutheniumkomplexen für die Krebstherapie diskutiert. Bei F. Kralik et al. Collection Czechoslov. Chem. Commun. 26 [1961] 1298 wird die Synthese von Komplexverbindungen des dreiwertigen Rutheniums mit Pyrazol und Imidazol beschrieben.

## Darstellung der Erfindung

Es wurde nun gefunden, daß die Komplexverbindungen der allgemeinen Formel I:

$$(BH_n)_m[RuX_{3+nm-q-r}(OH)_r(BH_p)_{3-nm+q}]_{1+p+q} \quad \text{I}$$

worin
B einen ein- oder mehrkernigen, einen oder mehrere Stickstoffatome enthaltenden basischen Heterocyclus,
X Chlor oder Brom,
m 1 oder 2,
n 1 oder 2, wobei die Summe aus n und m nicht größer als 3 ist,
p 0 oder 1, aber nicht 1, falls n 1 ist,
q 0 oder 1, aber nicht 1, falls p 1 ist und
r 0 oder 1,
bedeuten, eine vorteilhafte tumorhemmende Wirksamkeit bei günstiger Toxizität aufweisen. Sie eignen sich daher als Chemotherapeutikum zur Behandlung von Krebskrankheiten. Sie sind als nebenwirkungsarme chemotherapeutischen Mittel für die Behandlung von Tumoren, z. B. von ovariellen Tumoren, Mammatumoren, Magentumoren, Prostatatumoren, Lungentumoren, Blasentumoren und insbesondere colorectalen Tumoren und von anderen malignen Neoplasien geeignet. Dementsprechend sind die Verbindungen nützlich für die Erleichterung der mit der Krebstherapie verbundenen Schmerzen und Leiden, für die Hemmung und Rückbildung von Tumoren, für die Linderung der Symptome und die Erhöhung der Lebenserwartung.

Gegenstand der Erfindung sind daher Arzneimittelzubereitungen enthaltend diese Rutheniumkomplexverbindungen, und deren Verwendung zur Herstellung von Arzneimittelzubereitungenn, insbesondere von solchen gegen Krebskrankheiten und Verfahren zur Behandlung von krebskranken Lebewesen.

Besonders bevorzugt sind Arzneimittelzubereitungen enthaltend Komplexverbindungen der allgemeinen Formel I',

$$(B'H_n)_m[RuX'_{3+nm-q-r}(OH_r(B'H_p)_{3-nm+q}]_{1+p+q} \quad \text{I'},$$

worin
B' Pyridin, Pyrimidin, Pyridazin, Triazin, die durch Hydroxy, Amino, Halogen, $C_2$-$C_4$-Alkyl, $C_2$-$C_4$-Alkenyl, $C_1$-$C_4$-Alkylmercapto, Formyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamin-$C_1$-$C_4$-alkylen, Di-$C_1$-$C_4$-alkylaminocarbonyl, Di-$C_1$-$C_4$-alkylaminocarbonyl-$C_1$-$C_4$-alkylen, Hydroxyiminomethin, Phenyl, Benzyl, Benzoyl, Pyrrolidino, Piperidino, Pyrrol-1-yl oder Pyrrol-1-yl-$C_1$-$C_4$-alkylen substituiert sein können, oder einen Ring

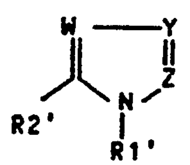

wobei
R1' Wasserstoff Natrium $C_1$-$C_4$-Alkyl oder Phenyl,

R2' Wasserstoff, $C_1$-$C_4$-Alkyl, Amino, Phenyl oder R1' und R2' zusammen eine Gruppe $-(CH_2)_s-$, wobei s für eine ganze Zahl von 4 bis 8 steht,

W Stickstoff oder CR3', wobei R3' Wasserstoff, $C_1$-$C_4$-Alkyl, Amino oder Phenyl, vorzugsweise Wasserstoff oder Methyl, bedeutet,

Y Stickstoff oder $CR_4'$, wobei $R_4'$ Wasserstoff, $C_1$-$C_4$-Alkyl, Amino oder Phenyl, vorzugsweise Wasserstoff oder Methyl, bedeutet,

Z Stickstoff oder CR5', wobei R5' Wasserstoff, $C_1$-$C_4$-Alkyl, Amino oder Phenyl, vorzugsweise Wasserstoff oder Methyl, bedeutet,

jedoch mindestens einer der Reste R3', R4' oder R5' Wasserstoff bedeutet,

Purin, Adenin, Guanin oder Cytosin, und

X' Chlor oder Brom bedeuten und

m, n, p, q und r die vorstehenden Bedeutungen haben.

Besonders bevorzugt sind Arzneimittelzubereitungen enthaltend Komplexverbindungen der allgemeinen Formel I''

$$(B''H_n)_m[RuX''_{3+nm-q-r}(OH)_r(B''H_p)_{3-nm+q}]_{1+p+q} \qquad I'',$$

worin

B'' Pyridin, das durch Hydroxy, Amino, Chlor, Diethylamino, Dimethylamino, Hydroxyiminomethin, Phenyl, Pyrrolidino, Piperidino oder Pyrrol-1-ylmethyl vorzugsweise in 4-Stellung substituiert sein kann, Pyrrol, Imidazol, 1-Methylimidazol, 4-Methylimidazol, Pyrazol, 4-Methylpyrazol, Triazol, 1-Natriumpyrazol, 1-Phenyltetrazol, 5-Phenyltetrazol, Adenin, Guanin, Purin oder Cytosin und

X'' Chlor bedeuten und

m, n, p, q und r die vorstehenden Bedeutungen haben.

Ganz besonders bevorzugt sind Arzneimittelzubereitungen enthaltend Imidazoliumdiimidazoltetrachlororuthenat(III), Bisimidazoliumimidazolpentachlororuthenat(III), Bispyrazoliumpyrazolhydroxotetrachlororuthenat(III) und/oder Bis(4-methylpyrazolium)(4-methylpyrazol)hydroxotetrachlororuthenat(III).

Die Verbindungen der allgemeinen Formel Ia,

$$(AH_n)_m[RuX_{3+nm-q-r}(OH)_r(AH)_p)_{3-nm+q}]_{1+p+q} \qquad Ia$$

worin

A einen ein- oder mehrkernigen, einen oder mehrere Stickstoffatome enthaltenden basischen Heterocyclus mit Ausnahme von Pyrazol und Imidazol und

X Chlor oder Brom, bedeuten und m, n, p, q und r die vorstehenden Bedeutungen haben, sind neu und daher ein weiterer Gegenstand der Erfindung.

Bevorzugt sind Verbindungen der allgemeinen Formel Ia',

$$(A'H_n)_m[RuX'_{3+nm-q-r}(OH)_r(A'H_p)_{3-nm+q}]_{1+p+q} \qquad Ia',$$

worin

A' Pyridin, Pyrimidin, Pyridazin, Triazin, die durch Hydroxy, Amino, Halogen, $C_1$-$C_4$-Alkyl, $C_2$-$C_4$-Alkenyl, $C_1$-$C_4$-Alkylmercapto, Formyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamin-$C_1$-$C_4$-alkylen, Di-$C_1$-$C_4$-alkylaminocarbonyl, Di-$C_1$-$C_4$-alkylaminocarbonyl-$C_1$-$C_4$-alkylen, Hydroxyiminomethin, Phenyl, Benzyl, Benzoyl, Pyrrolidino, Piperidino, Pyrrol-1-yl oder Pyrrol-1-yl-$C_1$-$C_4$-alkylen substituiert sein können, oder einen Ring

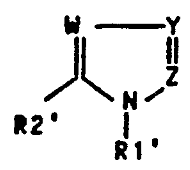

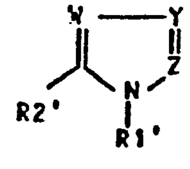

wobei

R1'Wasserstoff, Natrium $C_1$-$C_4$-Alkyl oder Phenyl,

R2' Wasserstoff, $C_1$-$C_4$-Alkyl, Amino, Phenyl oder R1' und R2' zusammen eine Gruppe $-(CH_2)-$, wobei s für

eine ganze Zahl von 4 bis 8 steht,
W Stickstoff oder CR3', wobei R3' Wasserstoff, $C_1$-$C_4$-Alkyl, Amino oder Phenyl, vorzugsweise Wasserstoff oder Methyl, bedeutet,
Y Stickstoff oder CR4', wobei R4' Wasserstoff, $C_1$-$C_4$-Alkyl, Amino oder Phenyl, vorzugsweise Wasserstoff oder Methyl, bedeutet,
Z Stickstoff oder CR5', wobei R5' Wasserstoff, $C_1$-$C_4$-Alkyl, Amino oder Phenyl, vorzugsweise Wasserstoff oder Methyl, bedeutet, jedoch mindestens einer der Reste R3', R4' oder R5' Wasserstoff bedeutet.
Purin, Adenin, Guanin oder Cytosin, aber nicht Pyrazol oder Imidazol, und
X' Chlor oder Brom bedeuten und
m, n, p, q und r die vorstehenden Bedeutungen haben.

Besonders bevorzugt sind die Verbindungen der allgemeinen Formel Ia'',

$$(A''H_n)_m[RuX''_{3+nm-q-r}(OH)_r(A''H_p)_{3-nm+q}]_{1+p+q} \qquad \text{Ia''},$$

worin
A'' Pyridin, das durch Hydroxy, Amino, Chlor, Diethylamino, Dimethylamino, Hydroxyiminomethin, Phenyl, Pyrrolidino, Piperidino oder Pyrrol-1-ylmethyl vorzugsweise in 4-Stellung substituiert sein kann, Pyrrol, 1-Methylimidazol, 4-Methylimidazol, 4-Methylpyrazol, Triazol, 1-Natriumpyrazol, 1-Phenyltetrazol, 5-Phenyltetrazol, Adenin, Guanin, Purin oder Cytosin und
X'' Chlor bedeuten und
m, n, p, q und r die vorstehenden Bedeutungen haben.

Insgesamt sind in den allgemeinen Formeln I, I', I'', Ia, Ia' bzw. Ia'' für A, A', A'', B, B' bzw. B'' 5-gliedrige Heterocyclen bevorzugt.

Die Herstellung der Rutheniumkomplexverbindungen erfolgt nach der bei F. Kralik et al., Collection Czechoslov. Chem. Commun. 26 [1961] 1298 angegebenen Verfahrensweise. Die Stickstoffheterocyclen werden zu einer Lösung von Ruthenium(III)chlorid bzw. -bromid gegeben. Das gewünschte Produkt scheidet sich ab oder wird durch Zugabe eines organischen Lösungsmittels oder Einengen der Reaktionslösung und/oder Abkühlen ausgefallt. Als Lösungsmittel für das Ruthenium(III) halogenid eignet sich beispielsweise ein Gemisch von Ethanol und wäßriger Halogenwasserstoffsäure. Vorzugsweise wird das Ruthenium(III) halogenid in einem 1 : 1-Gemisch von Ethanol und 1n wäßriger Halogenwasserstoffsäure gelöst. Es ist günstig, diese Lösung etwa 4 bis 16, vorzugsweise etwa 12 Stunden am Rückfluß zu sieden und anschließend auf ungefähr ein Viertel des Volumens einzuengen. Die basischen Heterocyclen werden zweckmäßigerweise gelöst in wäßriger Säure, vorzugsweise Halogenwasserstoffsäure zu der Ruthenium(III)halogenidlösung zugegeben. Beispielsweise werden die Heterocyclen gelöst in 6n wäßriger Halogenwasserstoffsäure zugegeben. Das Reaktionsgemisch wird bei Raumtemperatur belassen oder etwa eine halbe bis drei Stunden am Rückfluß erhitzt. Die Heterocyclen sind bekannt, oder können nach an sich bekannten Methoden hergestellt werden.

Gegenstand der Erfindung ist weiterhin ein Verfahren zur Herstellung der Verbindungen der allgemeinen Formel Ia, Ia' bzw. Ia'', worin A, A' bzw. A'', X, X' bzw. X'', m, n, p, q und r die vorstehend angegebenen Bedeutungen haben, das dadurch gekennzeichnet ist, daß man ein Ruthenium(III) halogenid der Formel $RuX_3$, $RuX'_3$ bzw. $RuX''_3$, worin X, X' bzw. X'' die vorstehend angegebenen Bedeutungen haben mit dem Stickstoffheterocyclus A, A' bzw. A'' der vorstehend angegebenen Bedeutung umsetzt.

Bevorzugt wird das erfindungsgemäße Verfahren in halogenwasserstoffsaurem Medium, insbesondere einem Gemisch eines niederen Alkanol, insbesondere Ethanol, mit wäßriger Halogenwasserstoffsäure, durchgeführt.

$C_1$-$C_4$-Alkyl ist geradkettig oder verzweigt, vorzugsweise geradkettig. $C_1$-$C_4$-Alkylen ist geradkettig oder verzweigt, beispielsweise Methylen, Ethylen, Propylen, Trimethylen und Tetramethylen, wobei geradkettig bevorzugt ist.

Die erfindungsgemäßen Arzneimittelzubereitungen werden vor allem intravenös, aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Die Arzneimittelzubereitungen werden nach an sich bekannten Verfahren hergestellt, wobei die erfindungsgemäßen Verbindungen als solche oder gegebenenfalls in Kombination mit geeigneten pharmazeutischen Trägerstoffen eingesetzt werden. Enthalten die neuen pharmazeutischen Zubereitungen neben dem Wirkstoff pharmazeutische Trägerstoffe, beträgt der Wirkstoffgehalt dieser Mischungen 0,1 bis 99,5, vorzugsweise 0,5 bis 95 Gewichtsprozent der Gesamtmischung.

In Obereinstimmung mit der Erfindung wird ein Wirkstoff in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis

entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäß der Erfindung können, wenn sie in Einheitsdosen vorliegen und für die Applikation z. B. am Menschen bestimmt sind, etwa 0,1 bis 500 mg, vorteilhafterweise 10 bis 200 mg und insbesondere 50 bis 150 mg Wirkstoff enthalten.

Im allgemeinen werden in der Humanmedizin der oder die Wirkstoffe bei oraler Gabe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht. Bei einer oralen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z. B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation der Arzneimittelzubereitungen sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Es kann sich auch als zweckmäßig erweisen, die Arzneimittelzubereitungen nur einmalig oder im Abstand von mehreren Tagen zu verabreichen.

Die Festlegung der jeweils erforderlichen optimalen Dosierung und Applikationsart der Wirkstoffe kann durch jeden Fachmann aufgrund seines Fachwissens erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z. B. Tabletten, Dragées, harte und weiche Kapseln, z. B. aus Gelatine, dispergierbare Pulver, Granulate, wäßrige und ölige Suspensionen, Emulsionen, Lösungen oder Sirupe kommen.

Tabletten können inerte Verdünnungsmittel, z. B. Calciumcarbonat, Calciumphosphat, Natriumphosphat oder Lactose; Granulierungs- und Verteilungsmittel, z. B. Maisstärke oder Alginate; Bindemittel, z. B. Stärke, Gelatine oder Akaziengummi; und Gleitmittel, z. B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption der Arzneimittelzubereitungen im Gastrointestinaltrakt bewirkt, so daß z. B. eine bessere Verträglichkeit, Protrahierung oder eine Retardierung erreicht wird. Gelatinekapseln können den Arzneistoff vermischt mit einem festen, z. B. Calciumcarbonat oder Kaolin, oder einem öligen. z. B. Oliven-, Erdnuß- oder Paraffinöl, Verdünnungsmittel enthalten.

Wäßrige Suspensionen, die gegebenenfalls kurzfristig zubereitet werden, können Suspendiermittel, z. B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z. B. Polyoxyethylenstearat, Heptadecaethylenoxycetanol, Polyoxyethylensorbitolmonooleat, Polyoxyethylensorbitanmonooleat oder Lecithin; Konservierungsmittel, z. B. Methyl- oder Propylhydroxybenzoate; Geschmacksmittel; Süßungsmittel, z. B. Saccharose, Lactose, Natriumcyclamat, Dextrose, Invertzuckersirup, enthalten.

Ölige Suspensionen können z. B. Erdnuß-, Oliven-, Sesam-, Kokos- oder Paraffinöl und Verdickungsmittel, wie z. B. Bienenwachs, Hartparaffin oder Cetylalkohol, enthalten; ferner Süßungsmittel, Geschmacksmittel und Antioxidantien.

In Wasser dispergierbare Pulver und Granulate können eine Rutheniumkomplexverbindung in Mischung mit Dispergier-, Benetzungs- und Suspendiermitteln, z. B. den oben genannten, sowie mit Süßungsmitteln, Geschmacksmitteln und Farbstoffen enthalten.

Emulsionen können z. B. Oliven-, Erdnuß- oder Paraffinöl neben Emulgiermitteln, wie z. B. Akaziengummi, Traganthgummi, Phosphatiden, Sorbitanmonooleat, Polyoxyethylensorbitanmonooleat, und Süßungs- und Geschmacksmitteln enthalten.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z. B. Propylen- oder Butylenglykol, und/oder Lösungsvermittler, z. B. Tweene®, Cremophore® oder Polyvinylpyrrolidon, enthalten.

Die Wirkstoffe können auch in Form von Kopräzipitaten mit hydrophilen Polymeren eingesetzt werden. Kopräzipitate können hergestellt werden, indem man Lösungen der Komplexverbindungen in einem wasserfreien organischen Lösungsmittel mit Lösungen von hydrophilen Polymeren, wie beispielsweise Polyvinylpyrrolidon (PVP) oder Polyoxyethylensorbitanfettsäureestern (Tween®) oder insbesondere Glycerin-

Polyethylenglycolricinoleat (Cremophor®EL) vermischt. Die nach Abziehen des bzw. der Lösungsmittel zurückbleibenden Rückstände werden als wäßrige Lösungen verabreicht. Als organische Lösungsmittel kommen beispielsweise Chloroform oder Methylenchlorid in Frage, die vor der Verwendung auf übliche Weise wasserfrei gemacht werden. Es hat sich als zweckmäßig erwiesen, die hydrophilen Polymeren in einem 5- bis 50-, vorzugsweise 10- bis 35-fachen gewichtsmäßigen Überschuß gegenüber der Komplexverbindung anzuwenden. Es ist auch möglich, die Polymeren als solche in Lösungen der Komplexverbindungen einzutragen. Den nach Abziehen des bzw. der Lösungsmittel verbleibenden Rückstand befreit man zweckmäßigerweise im Hochvakuum möglichst weitgehend von Lösungsmittelresten. Man erhält je nach Art und Menge des verwendeten hydrophilen Polymeren feste kristallartige oder glasartige oder auch flüssige oder klebrige Rückstände. Letztere lassen sich in der Regel durch Abkühlung in feste, meist wachsartige Produkte überführen. Günstig auf das Löseverhalten der Kopräzipitate wirkt es sich aus, wenn man bei deren Herstellung zu dem Gemisch der Lösungen der Komplexverbindung und des hydrophilen Polymeren noch Dispergier- oder Benetzungsmittel, wie Propylen- oder Butylenglycol, vorzugsweise Propylenglycol, zugibt.

Die Wirkstoffe können gegebenenfalls mit einem oder mehreren der angegebenen Träger- oder Zusatzstoffe auch in mikroverkapselter Form formuliert werden.

Die folgenden Beispiele erläutern die Erfindung näher ohne sie einzuschränken.

## Herstellungsbeispiele

### 1. Allgemeine Vorschrift

In einer Mischung aus 250 ml Ethanol und 250 ml wäßriger in Salzsäure werden 10 g (38,3 mmol) Ruthenium(III) chloridtrihydrat gelöst. Die Lösung wird 12 Stunden am Rückfluß gekocht und anschließend auf ein Volumen von 125 ml eingeengt. Diese Lösung enthält 0,3 mmol Ruthenium pro ml und wird nachstehend als "Ruthenium-Lösung" bezeichnet.

Die Lösung wird mit salzsauren Lösungen des basischen Stickstoffheterocyclus versetzt.

### 2. Detail-Vorschriften

2.1. Di(1,2,4-triazolium)pentachloro(1,2,4-triazol)ruthenat(III)

$(C_2H_4N_3)_2[RuCl_5(C_2H_3N_3)]$

Zu 8,9 ml der Ruthenium-Lösung werden 0,93 g 1,2,4-Triazol, gelöst in 7,2 ml 6n Salzsäure, gegeben. Am Rotationsverdampfer wird auf zwei Drittel des ursprünglichen Volumens eingeengt und auf 4°C abgekühlt. Nach 12 Stunden wird ein brauner Niederschlag abfiltriert.

Schmelzpunkt: >300°C.
Ausbeute: 0,55 g (50 % d.Th.)
Analyse: Ber.: 14,76 % C 2,25 % H 25,84 % N

Gef.: 14,45 % C 2,54 % H 25,15 % N

2.2. (1,2,4-Triazolium)tetrachlorodi(1,2,4-Triazol)Ruthenat(III)

$(C_2H_4N_3)[RuCl_4(C_2H_3N_3)_2]$

Zu 19,7 ml der Ruthenium-Lösung werden 5,8 g 1,2,4-Triazol, gelöst in 16,6 ml 6n Salzsäure, gegeben. Anschließend wird eine Stunde bei Zimmertemperatur gerührt und dann das Volumen der Lösung auf die Hälfte reduziert. Dabei beginnt sich ein dunkelbrauner Niederschlag zu bilden und nach Abkühlen auf 4°C kann das Produkt abgesaugt werden.

Schmelzpunkt: 179°C

Ausbeute:
1,5 g (56,3 % d.Th.)
Analyse: Ber.: 15,97 % C 2,23 % H 27,95 % N
Gef.: 16,92 % C 2,81 % H 28,66 % N

2.3. Adenindiumdi[adeniniumpentachlororuthenat(III)]tetrahydrat

$(C_5H_7N_5)[RuCl_5(C_5H_6N_5)]_2 \times 4\ H_2O$

1,5 g (11 mmol) Adenin werden in 100 ml 4n Salzsäure gelöst und mit 10 ml der vorbereiteten Ruthenium-Lösung versetzt. Man engt am Rotationsverdampfer etwas ein und stellt die Lösung in den Kühlschrank. Nach 12 Stunden kann ein brauner Niederschlag abgesaugt werden.

Schmelzpunkt: > 300° C
Ausbeute: 0,8 g (51,6 % d.Th.)
Analyse: Ber.: 17,35 % C 2,62 % H 20,23 % N
Gef.: 17,81 % C 2,75 % H 20,29 % N

2.4. Guanindiumpentachloroguaninruthenat(III)dihydrat

$(C_5H_7N_5O)[RuCl_5(C_5H_5N_5O)] \times 2\ H_2O$

3 g (19,8 mmol) Guanin werden in 100 ml heißer 4n Salzsäure gelöst und zu 7,5 ml der Ruthenium-Lösung getropft. Es wird eine Stunde unter Rückfluß gekocht und danach der kaffebraune Niederschlag heiß abgenutscht.

Schmelzpunkt: > 300° C
Ausbeute: 0,78 g (56,1 % d.Th.)
Analyse: Ber.: 19,41 % C 2,61 % H 22,64 % N
Gef.: 19,92 % C 2,57 % H 22,57 % N

2.5. Dicytosinium[pentachlorocytosinruthenat(III)]pentahydrat

$(C_4H_6N_3O)_2[RuCl_5(C_4H_5N_3O)] \times 5\ H_2O$

1 g (9 mmol) Cytosin wird in 50 ml heißer 4n Salzsäure gelöst und mit 3 ml der Ruthenium-Lösung versetzt. Nach einer Nacht im Kühlschrank fällt ein rotbrauner Niederschlag aus, der nach dem Absaugen im Hochvakuum getrocknet wird.

Schmelzpunkt: 190 - 195° C
Ausbeute: 0,21 (41,2 % d.Th.)
Analyse: Ber.: 20,46 % C 3,83 % H 17,90 % N
Gef.: 21,52 % C 3,17 % H 18,29 % N

2.6. Purindiumpurinpentachlororuthenat(III)

$(C_5H_6N_4)[RuCl_5(C_5H_4N_4)]$

1,4 g (11,7 mmol] Purin werden in 3 ml 6n Salzsäure gelöst. Die Lösung wird mit 4 ml der Ruthenium-Lösung versetzt. Man erhält einen kaffeebraunen Niederschlag.

Schmelzpunkt: >300° C
Ausbeute: 0,39 g (62,1 % d.Th.)
Analyse: Ber.: 23,07 % C 1,94 % H 21,53 % N
Gef.: 23,91 % C 2,07 % H 22,27 % N

2.7. Bis(1-methylimidazolium)pentachloro-1-methylimidazolruthenat(III)

$(C_4H_7N_2)_2[RuCl_5(C_4H_6N_2)]$

2 g (24 mmol) 1-Methylimidazol werden in 4 ml 6n Salzsäure gelöst und zu 10 ml der Ruthenium-Lösung gegeben. Das Produkt wird durch Zugabe von Ethanol ausgefällt.

Schmelzpunkt: 150 - 153° C (Verfärbung bei 75° C)
Ausbeute: 1,2 g (75,9 % d.Th.)
Analyse: Ber.: 27,36 % C 4,21 % H
Gef: 27,50 % C 4,01 % H

2.8. Bis[1-Natrium(1,2,4-triazolium)]pentachloro[1-natrium(1,2,4-triazol]ruthenat(III)

$(C_2H_3N_3Na)_2[RuCl_5(C_2H_2N_3Na)_2]$

Man löst 2,67 g 1-Natrium(1,2,4-triazol) in 5 ml Wasser, gibt 0,5 ml 6n Salzsäure dazu und versetzt mit 10 ml der Ruthenium-Lösung. Beim 12-stündigen Stehenlassen fallen rotbraune Kristalle aus.

Schmelzpunkt: 120° C
Ausbeute: 1,2 g (71,9 % d.Th.)
Analyse: Ber.: 13,94 % C 1,45 % H 22,65 % N
Gef: 12,50 % C 2,26 % H 21,68 % N

2.9. 4-Methylimidazoliumtetrachlorobis(4-methylimidazol)ruthenat(III)

$(C_4H_7N_2)[RuCl_4(C_4H_6N_2)_2]$

Man löst 1,21 g (14,7 mmol) 4-Methylimidazol in 0,6 ml 6n Salzsäure und gibt 5 ml der Ruthenium-Lösung zu. Es wird dann auf ungefähr zwei Drittel des ursprünglichen Volumens eingeengt und die Lösung über Nacht in den Kühlschrank gestellt. Nach 12 Stunden ist das Produkt in Form von dunkelroten Kristallen ausgefallen. Es wird abgesaugt und mit Diethylether gewaschen.

Schmelzpunkt: 223° C (Substanz beginnt bei 181° C zähflüssig zu werden)
Ausbeute: 0,49 g (66,6 % d.Th.)
Analyse: Ber.: 29,34 % C 3,90 % H 17,14 % N
Gef.: 29,44 % C 4,07 % H 17,19 % N

2.10. 1,5-Dimethyltetrazoldiumbis[pentachloro-1,5-dimethyltetrazoliumruthenat(III)]hydrat

$(C_3H_8N_4)[RuCl_5(C_3H_7N_4)]_2 \times H_2O$

Es werden 1,44 g 1,5-Dimethyltetrazol in wenig Wasser und 0,5 ml 6n Salzsäure gelöst und mit 5 ml der Ruthenium-Lösung versetzt. Dann wird das Volumen auf etwa zwei Drittel des ursprünglichen eingeengt und im Kühlschrank auf 4° C abgekühlt. Nach 12 Stunden kann das gelbe Produkt abgesaugt werden.

Schmelzpunkt: >280° C
Ausbeute: 0,51 g (71 % d.Th.)
Analyse: Ber.: 16,55 % C 3,49 % H 25,75 % N
Gef: 16,23 % C 2,81 % H 25,22 % N

## Pharmakologie

**A. P 388 Leukämie-Modell**

Ca. 4 Wochen alten 18 bis 20 g schweren weiblichen $BDF_1$-Mäusen werden ca. 2 x $10^5$ oder ca. $10^6$ P 388-Leukämiezellen in 0,2 ml physiologischer Kochsalzlösung intraperitoneal (i.p.) übertragen. Die Leukämie wird auf DBA/2-Mäusen in Passage gehalten. Die Leukämiezellen werden frisch getöteten Tieren unmittelbar vor der Transplantation entnommen. Beim Überimpfen werden die Tiere randomisiert. Pro Dosierung werden 3 bis 6 Mäuse verwendet. Die Anzahl der Kontrollgruppen (unbehandelte Tiere) wird bei umfangreicheren Versuchen so gewählt, daß sie in etwa der Quadratzahl aus der Gesamtzahl der Gruppen entspricht. Die Substanzen werden als wäßrige Lösungen, falls notwendig unter Zuhilfenahme von Lösungsvermittlern, beispielsweise Tween® (Polyoxyethylenderivate von Sorbitanestern), erstmals 24 Stunden nach der Transplantation intraperitoneal gespritzt. Die Versuchsbedingungen entsprechen dem P 388-Leukämie-Modell des US-amerikanischen National Cancer Institute (NCI). (Methods of Development of New Anticancer Drugs, NCI Monography 45, US Department of Health, Education and Wellfare, Public Health Service, März 1977, Seite 147).

**2. Leukämie 1210-Modell**

Die Durchführung erfolgt in Analogie zum unter A.1 beschriebenen P 388-Leukämie-Modell. Es werden $10^5$ L 1210-Leukämiezellen übertragen. Wie beim Modell des NCI wird die Substanz nur einmal appliziert (NCI Monography 45, Seite 147).

**3. Melanom B16-Modell**

Die Durchführung erfolgt in Analogie zum unter A.1 beschriebenen P 388-Leukämie-Modell nach der in der NCI-Monography 45, Seite 148 angegebenen Vorgehensweise. Der Tumor wird als Homogenat (0,5 ml des in der NCI Monography beschriebenen Homogenats) subcutan (s.c.) übertragen. Therapie erfolgt an den Tagen 1 bis 9. Am Tag 22 wird das Tumorgewicht bestimmt.

**4. AMMN-industriertes autochthones Colonkarzinom**

Die Methode ist beschrieben bei J. Wagner et al., J. Cancer Res. Clin. Oncol. 101 [1985] 115 - 131. Der Colontumor wird an männlichen Sprague-Dawley Ratten durch zehnmalige intrarectale Applikation von 2 mg/kg/Woche von N-Nitrosoacetoxymethyl-methylamin (AMMN) induziert. Nach der zehnwöchigen Induktionsperiode wird der Tumor fünf Wochen wachsen gelassen. Dann erfolgt die zehnwöchige Therapiephase mit zweimaliger wöchentlicher Verabreichung der Testsubstanzen. Eine Woche nach Therapie ende werden die Tiere getötet und die Tumorvolumen bestimmt.

**B. Versuchsergebnisse**

In der nachfolgenden Tabelle sind Ergebnisse aus den unter Punkt A.1, 2, und 3, beschriebenen Tumormodellen zusammengestellt. Die angegebene Dosis wurde an den in der dritten Spalte der Tabelle aufgeführten Tagen appliziert. Der in Prozent angegebene Faktor T/C bedeutet die prozentuale Verlängerung der medianen Oberlebenszeit der behandelten Tiere im Vergleich zur medianen Oberlebenszeit der unbehandelten Kontrolltiere.

Der Versuch wird abgebrochen, sobald die mediane Überlebenszeit T/C der behandelten Tiere 300 % der medianen Oberlebenszeit der unbehandelten Tiere erreicht hat. Zur Berechnung der medianen Oberlebenszeit werden die bei Versuchsende noch lebenden Tiere als bei Versuchsende gestorben berücksichtigt.

Die untersuchten Verbindungen sind durch eine laufende Nummer (1fd.Nr.) bezeichnet:

1 = Bisimidazoliumimidazolpentachlororuthenat(III)
2 = Imidazoliumdiimidazoltetrachlororuthenat(III)
3 = Bispyrazoliumpyrazolhydroxotetrachlororuthenat(III).
4 = Bis(4-methylpyrazolium)(4-methylpyrazol)hydroxotetrachlororuthenat(III)
5 = Bis(4-methylimidazolium)(4-methylimidazol)pentachlororuthenat(III)
6 = 2-Methylimidazolium(2-methylimidazol)tetrachlororuthenat(III)
7 = Bis(2-methylimidazolium)(2-methylimidazol)pentachlororuthenat(III)
8 = Di(1,2,4-triazolium)pentachloro(1,2,4-triazol)ruthenat(III)
9 = Purindiumpurinpentachlororuthenat(III)
10 = Dicytosiniumpentachlorocytosinruthenat(III)pentahydrat
11 = Bis(1-methylimidazolium)(1-methylimidazol)pentachlororuthenat(III)
12 = Bispyrimidiniumpentachloropyrimidinruthenat(III)
13 = Adenindiumdi[adeniniumpentachlororuthenat(III)]tetrahydrat
14 = 4-Methylimidazoliumtetrachlorobis(4-methylimidazol)ruthenat(III)
15 = 1,2,4-Triazoliumtetrachlorobis(1,2,4-triazol)ruthenat(III)

| Verbindung Lfd.Nr. | Zahl der Leukämie-zellen | Tage, an denen therapiert wird | Dosis [mg/kg] | T/C [%] P 388 | L 1210 |
|---|---|---|---|---|---|
| Fluoruracil | $10^6$ | 1, 5, 9 | 60 | 131 | |
| 1 | $10^6$ | 1 | 78 | 125 | |
| | $10^6$ | 1 bis 9 | 39 | 170 | |
| | $10^5$ | 1 | 78 | | 131 |
| | $10^6$ | 1, 5, 9 | 39 | 200 | |
| 2 | $10^6$ | 1 | 72 | 181 | |
| | $10^6$ | 1 bis 9 | 8 | 181 | |
| | $2 \times 10^5$ | 1, 5, 9 | 36 | 186 | |
| 3 | $10^6$ | 1 | 75 | 149 | |
| | $10^6$ | 1 bis 9 | 8 | 173 | |
| | $2 \times 10^5$ | 1, 5, 9 | 28 | 142 | |
| 4 | $10^6$ | 1, 5, 9 | 10 | 180 | |
| 5 | $10^6$ | 1, 5, 9 | 48,9 | 149 | |
| 6 | $10^6$ | 1, 5, 9 | 48,9 | 127 | |
| 7 | $10^6$ | 1, 5, 9 | 48,9 | 138 | |
| 8 | $10^6$ | 1, 5, 9 | 45,1 | 161 | |
| | $10^6$ | 1, 5, 9 | 83,3 | >250 | |
| 9 | $10^6$ | 1, 5, 9 | 52,1 | 135 | |
| 10 | $10^6$ | 1, 5, 9 | 104,3 | 147 | |
| 11 | $10^6$ | 1, 5, 9 | 52,4 | 120 | |
| 12 | $10^6$ | 1, 5, 9 | 52,0 | 143 | |
| 13 | $10^6$ | 1, 5, 9 | 103,7 | 141 | |
| 14 | $10^6$ | 1, 5, 9 | 48,9 | 120 | |
| 15 | $10^6$ | 1, 5, 9 | 45,1 | 161 | |

Die Auswertung der Ergebnisse aus dem Melanom B 16-Modell ergab, daß die Verbindung mit der laufenden Nummer 1 bei den therapierten Tieren bewirkte, daß deren Tumorgewicht nur 10 % der Tumorgewichte der Kontrolltiere betrug.

Die Auswertung der Ergebnisse aus dem AMMN-induzierten autochthonen Colonkarzinom-Modell ergab, daß die Verbindung mit der laufenden Nummer 1 (Dosierung pro Verabreichung: 12 mg/kg) bei den therapierten Tieren bewirkte, daß deren Tumorvolumina nur 20 % der Tumorvolumina der unbehandelten Kontrolltiere betrugen. Dies ist um so erstaunlicher, als die klassischen in der Klinik angewandten Cytostaticas, wie beispielsweise Endoxan oder Cisplatin, an diesem Modell unter den beschriebenen Bedingungen keine therapeutische Wirkung zeigen. Nachdem dieses Modell als besonders aussagekräftig im Hinblick auf die

Wirksamkeit getesteter Verbindungen bei der Behandlung von Colontumoren und colorectalen Tumoren beim Menschen gilt, kann mit großer Wahrscheinlichkeit davon ausgegangen werden, daß die erfindungsgemäßen Arzneimittelzubereitungen bei der Behandlung von Colontumoren und colorectalen Tumoren beim Menschen von Nutzen sind.

**Patentansprüche**

1. Verbindungen der allgemeinen Formel I

$(BH_n)_m[RuX_{3+nm-q-r}(OH)_r(BH_p)_{3-nm+q}]_{1+p+q}$ I,

worin
B einen ein- oder mehrkernigen, einen oder mehrere Stickstoffatome enthaltenden basischen Heterocyclus,
X Chlor oder Brom,
m 1 oder 2,
n 1 oder 2, wobei die Summe aus n und m nicht größer als 3 ist,
p 0 oder 1, aber nicht 1, falls n 1 ist,
q 0 oder 1, aber nicht 1, falls p 1 ist und
r 0 oder 1, bedeuten, zur Verwendung in einem therapeutischen Verfahren.

2. Verbindungen der allgemeinen Formel I',

$(B'H_n)_mRuX'_{3+nm-q-r}(OH)_r(B'H_p)_{3-nm+q}]_{1+p+q}$ I',

worin
B' Pyridin, Pyrimidin, Pyridazin, Triazin, die durch Hydroxy, Amino, Halogen, $C_1$-$C_4$-Alkyl, $C_2$-$C_4$-Alkenyl, $C_1$-$C_4$-Alkylmercapto, Formyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamin-$C_1$-$C_4$-alkylen, Di-$C_1$-$C_4$-alkylaminocarbonyl, Di-$C_1$-$C_4$-alkylaminocarbonyl-$C_1$-$C_4$-alkylen, Hydroxyiminomethin, Phenyl, Benzyl, Benzoyl, Pyrrolidino, Piperidino, Pyrrol-1-yl oder Pyrrol-1-yl-$C_1$-$C_4$-alkylen substituiert sein können oder einen Ring
wobei

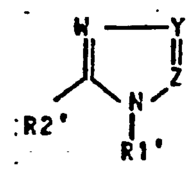

wobei
R1' Wasserstoff, Natrium, $C_1$-$C_4$-Alkyl oder Phenol,
R2' Wasserstoff, $C_1$-$C_4$-Alkyl, Amino, Phenyl oder R1' und R2' zusammen eine Gruppe $(CH_2)_s$-, wobei s für eine ganze Zahl von 4 bis 8 steht,
W Stickstoff oder CR3', wobei R3' Wasserstoff, $C_1$-$C_4$-Alkyl, Amino oder Phenyl, vorzugsweise Wasserstoff oder Methyl, bedeutet,
Y Stickstoff oder CR4', wobei R4' Wasserstoff, $C_1$-$C_4$-Alkyl, Amino oder Phenyl, vorzugsweise Wasserstoff oder Methyl, bedeutet,
Z Stickstoff oder CR5', wobei R5' Wasserstoff, $C_1$-$C_4$-Alkyl, Amino oder Phenyl, vorzugsweise Wasserstoff oder Methyl, bedeutet, jedoch mindestens einer der Reste R3', R4'oder R5' Wasserstoff bedeutet,
Purin, Adenin, Guanin oder Cytosin und
X' Chlor oder Brom bedeuten und
m, n, p, q und r die in Anspruch 1 angegebenen Bedeutungen haben, zur Verwendung in einem therapeutischen Verfahren.

3. Verbindungen der allgemeinen Formel I''

$(B''H_n)_m[RuX''_{3+nm-q-r}(OH)_r(B''H_p)_{3-nm+q}]_{1+p+q}$ I'',

worin
B'' Pyridin, das durch Hydroxy, Amino, Chlor, Diethylamino, Dimethylamino, Hydroxyiminomethin, Phenyl, Pyrrolidino, Piperidino oder Pyrrol-1-ylmethyl, vorzugsweise in 4-Stellung, substituiert sein kann, Pyrrol, Imidazol, 1-Methylimidazol,
4-Methylimidazol, Pyrazol, 4-Methylpyrazol, Triazol, 1-Natriumpyrazol, 1-Phenyltetrazol, 5-Phenyltetrazol, Adenin, Guadin, Purin oder Cytosin und
X'' Chlor bedeuten und
m, n, p, q und r die in Anspruch 1 angegebenen Bedeutungen haben, zur Verwendung in einem therapeutischen Verfahren.

4. Bisimidazoliumimidazolpentachlororuthenat(III), Imidazoliumdiimidazoltetrachlororuthenat(III), Bispyrazoliumpyrazolhydroxotetrachlororuthent(III), Bis(4-methylpyrazolium)(4-methylpyrazol)hydroxotetrachlororuthenat(III), zur Verwendung in einem therapeutischen Verfahren.

5. Komplexverbindungen der allgemeinen Formel Ia,

$$(AH_n)_m[RuX_{3+nm-q-r}(OH)_r(AH_p)_{3-nm+q}]_{1+p+q} \qquad \text{Ia,}$$

worin

A einen ein- oder mehrkernigen, einen oder mehrere Stickstoffatome enthaltenden basischen Heterocyclus mit Ausnahme von Pyrazol und Imidazol und

X Chlor oder Brom bedeuten und

m, n, p, q, und r die in Anspruch 1 angegebenen Bedeutungen haben.

6. Komplexverbindung der allgeimenen Formel Ia',

$$(A'H_n)_m[RuX'_{3+nm-q-r}(OH)_r (A'H_p)_{3-nm+q}]_{1+p+q} \qquad \text{Ia',}$$

worin

A' Pyridin, Pyrimidin, Pyridazin, Triazin die durch Hydroxy, Amino, Halogen, $C_1$-$C_4$-Alkyl, $C_2$-$C_4$-Alkenyl, $C_1$-$C_4$-Alkylmercapto, Formyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamin-$C_1$-$C_4$-alkylen, Di-$C_1$-$C_4$-alkylaminocarbonyl, Di-$C_1$-$C_4$-alkylaminocarbonyl-$C_1$-$C_4$-alkylen, Hydroxyiminomethin, Phenyl, Benzyl, Benzol, Pyrrolidino, Piperidino, Pyrrol-1-yl oder Pyrrol-1-yl-$C_1$-$C_4$-alkylen substituiert sein können oder einen Ring

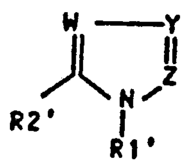

wobei

R1' Wasserstoff, Natrium, $C_1$-$C_4$-Alkyl oder Phenyl,

R2' Wasserstoff, $C_1$-$C_4$-Alkyl, Amino, Phenyl oder R2' und R' zusammen eine Gruppe $-(CH_2)_s-$, wobei s für eine ganze Zahl von 4 bis 8 steht,

W Stickstoff oder CR3', wobei R3' Wasserstoff, $C_1$-$C_4$-Alkyl, Amino oder Phenyl, vorzugsweise Wasserstoff oder Methyl, bedeutet,

Y Stickstoff oder CR4', wobei R4' Wasserstoff, $C_1$-$C_4$-Alkyl, Amino oder Phenyl, vorzugsweise Wasserstoff oder Methyl, bedeutet,

Z Stickstoff oder CR5', wobei R5' Wasserstoff, $C_1$-$C_4$-Alkyl, Amino oder Phenyl, vorzugsweise Wasserstoff oder Methyl, bedeutet, jedoch mindestens einer der Reste R3', R4'oder R5' Wasserstoff bedeutet,

Purin, Adenin, Guanin oder Cytosin, aber nicht Pyrazol oder Imidazol und

X' Chlor oder Brom bedeuten und

m, n, p, q und r die in Anspruch 1 angegebenen Bedeutungen haben.

7. Komplexverbindungen der allgemeinen Formel Ia'',

$$(A''H_n)_m[R_uX''_{3+mn-q-r}(OH)_r(A''H_p)_{3-nm+q}]_{1+p+q} \qquad \text{Ia''}$$

worin

A'' Pyridin, das durch Hydroxy, Amino Chlor, Diethylamino, Dimethylamino, Hydroxyiminomethin, Phenyl, Pyrrolidino, Piperidino oder Pyrrol-1-ylmethyl vorzugsweise in 4-Stellung substituiert sein kann, Pyrrol, 1-Methylimidazol, 4-Methylimidazol, 4-Methylpyrazol, Triazol, Natriumpyrazol, 1-Phenyltetrazol, 5-Phenyltetrazol, Adenin, Guanin, Purin oder Cytosin und

X'' Chlor bedeuten und

m, n, p, q und r die in Anspruch 1 angegebenen Bedeutungen haben.

8. Verfahren zur Herstellung nach Ansprüchen 5, 6 oder 7, dadurch gekennzeichnet, daß man ein Ruthenium(III) halogenid der Formel $RuX_3$, $RuX'_3$ beziehungsweise $RuX''_3$, worin X, X' beziehungsweise X'', die in den Ansprüchen 5, 6 beziehungsweise 7 angegebenen Bedeutungen haben mit dem Stickstoffheterocyclus A, A' beziehungsweise A'' der in den Ansprüchen 5, 6 beziehungsweise 7 angegebenen Bedeutungen umsetzt.

9. Verfahren nach Anspruch 8, dadurch gekennzeichnet, daß man die Umsetzung in halogenwasserstoffsaurem Medium durchführt.

10. Verfahren nach Anspruch 9, dadurch gekennzeichnet, daß man die Umsetzung in einem Gemisch eines niederen Alkanols mit wäßriger Halogenwasserstoffsäure durchführt.

11. Arzneimittelzubereitungen enthaltend eine mehrere Komplexverbindung nach einem der Ansprüche 5 bis 7.

12. Sterile wäßrige Lösungen enthaltend eine oder mehrere Komplexverbindungen nach einem der Ansprüche 1 bis 7.

13. Verwendung der in den Ansprüchen 1 bis 7 angegebenen Verbindungen zur Herstellung von Arzneimittelzubereitungen zur Behandlung von Krebskrankheiten.

14. Arzneimittelzubereitung umfassend einen pharmazeutisch annehmbaren Trägerstoff und 0,1 bis 99,5 Gewichtsprozent von mindestens einer in den Ansprüchen 1 bis 4 genannten Komplexverbindungen.

## Claims

1. Compounds corresponding to general formula I

$(BH_n)_m[RuX_{3+nm-q-r}(OH)_r(BH_p)_{3-nm+q}]_{1+p+q}$

in which
B is a mononuclear or polynuclear basic heterocycle containing one or more nitrogen atoms,
X is chlorine or bromine,
m is 1 or 2,
n is 1 or 2, the sum of n and m being no greater than 3,
p = 0 or 1, but not 1 where n is 1,
q is 0 or 1, but not 1 where p is 1 and
r is 0 or 1, for use in a therapeutic process.

2. Compounds corresponding to general formula I'

$(B'H_n)_m[RuX'_{3+nm-q-r}(OH)_r(B'H_p)_{3-nm+q}]_{1+p+q}$ I'

in which
B' is pyridine, pyrimidine, pyridazine, triazine, which may be substituted by hydroxy, amino, halogen, $C_{1-4}$ alkyl, $C_{2-4}$ alkenyl, $C_{1-4}$ alkylmercapto, formyl, $C_{1-4}$ alkoxycarbonyl, $C_{1-4}$-alkoxycarbonyl-$C_{1-4}$-alkylene, di-$C_{1-4}$-alkylamino, di-$C_{1-4}$-alkylamino-$C_{1-4}$-alkylene, di-$C_{1-4}$-alkylaminocarbonyl, di-$C_{1-4}$-alkylaminocarbonyl-$C_{1-4}$-alkylene, hydroxyiminomethine, phenyl, benzyl, benzoyl, pyrrolidino, piperidino, pyrrol-1-yl or pyrrol-1-yl-$C_{1-4}$-alkylene, or a ring

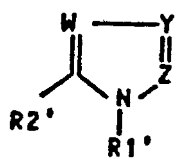

in which
R1, is hydrogen sodium $C_{1-4}$ alkyl or phenyl,
R2' is hydrogen, $C_{1-4}$ alkyl, amino, phenyl or
R1'and R2' together represent a group $-(CH_2)_s-$, where s is an integer of from 4 to 8,
W is nitrogen or CR3', where R3' is hydrogen, $C_{1-4}$ alkyl, amino or phenyl, preferably hydrogen or methyl,
Y is nitrogen or CR4', where R4' is hydrogen, $C_{1-4}$ alkyl, amino or phenyl, preferably hydrogen or methyl,
Z is nitrogen or CR5', where R5' is hydrogen, $C_{1-4}$ alkyl, amino or phenyl, preferably hydrogen or methyl, but at least one of the substituents R3', R4' or R5' is hydrogen,
purine, adenine, guanine or cytosine and
X' is chlorine or bromine and
m, n, p, q and r are as defined in claim 1, for use in a therapeutic process.

3. Compounds corresponding to general formula I''

$(B''H_n)_m[RuX''_{3+nm-q-r}(OH)_r(B''H_p)_{3-nm+q}]_{1+p+q}$ I''

in which
B'' is pyridine which may be substituted by hydroxy, amino, chlorine, diethylamino, dimethylamino, hydroxyaminomethine, phenyl, pyrrolidino, piperidino or pyrrol-1-ylmethyl, preferably in the 4-position, pyrrole, imidazole, 1-methylimidazole, 4-methylimidazole, pyrazole, 4-methylpyrazole, triazole, 1-sodium pyrazole, 1-phenyltetrazole, 5-phenyltetrazole, adeine, guanine, purine or cytosine,
X'' is chlorine and
m, n, p, q and r are as defined in claim 1, for use in a therapeutic process.

4. Bis-imidazolium imidazole pentachlororuthenate(III), imidazolium diimidazole tetrachlororuthenate(III), bispyrazolium pyrazole hydroxotetrachlororuthenate(III), bis-(4-methylpyrazolium)(4-methylpyrazole)hydroxotetrachlororuthenate(III) for use in a therapeutic process.

5. Complex compounds corresponding to general formula Ia

$(AH_n)_m[RuX_{3+nm-q-r}(OH)_r(AH_p)_{3-nm+q}]_{1+p+q}$ Ia

in which

A is a mononuclear or polynuclear basic heterocycle containing one or more nitrogen atoms, except for pyrazole and imidazole,

X is chlorine or bromine and

m, n, p, q and r are as defined in claim 1.

6. A complex compound corresponding to general formula Ia,

$(A'H_n)_m[RuX'_{3+nm-q-r}(OH)_r(A'H_p)_{3-nm+q}]_{1+p+q}$ Ia'

A' is pyridine, pyrimidine, pyridazine, triazine, which may be substituted by hydroxy, amino, halogen, $C_{1-4}$ alkyl, $C_{2-4}$ alkenyl, $C_{1-4}$ alkylmercapto, formyl, $C_{1-4}$ alkoxycarbonyl, $C_{1-4}$-alkoxycarbonyl-$C_{1-4}$-alkylene, di-$C_{1-4}$-alkylamino, di-$C_{1-4}$-alkylamino-$C_{1-4}$-alkylene, di-$C_{1-4}$-alkylaminocarbonyl, di-$C_{1-4}$-alkylaminocarbonyl-$C_{1-4}$-alkylene, hydroxyiminomethine, phenyl, benzyl, benzoyl, pyrrolidino, piperidino, pyrrol-1-yl or pyrrol-1-yl-$C_{1-4}$-alkylene, or a ring

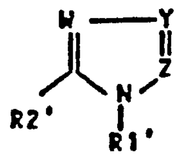

in which

R1' is hydrogen, sodium, $C_{1-4}$ alkyl or phenyl,

R2' is hydrogen $C_{14}$ alkyl amino phenyl or

R1' and R2' together represent a group $-(CH_2)_s-$, where s is an integer of from 4 to 8,

W is nitrogen or CR3', where R3' is hydrogen, $C_{1-4}$ alkyl, amino or phenyl, preferably hydrogen or methyl,

Y is nitrogen or CR4', where R4' is hydrogen, $C_{1-4}$ alkyl, amino or phenyl, preferably hydrogen or methyl,

Z is nitrogen or CR5', where R5' is hydrogen, $C_{1-4}$ alkyl, amino or phenyl, preferably hydrogen or methyl, but at least one of the substituents R3', R4' or R5' is hydrogen,

purine, adenine, guanine or cytosine, but not pyrazole or imidazole,

X' is chlorine or bromine and

m, n, p, q and r are as defined in claim 1.

7. Complex compounds corresponding to general formula Ia''

$(A''H_n)_m[RuX''_{3+nm-q-r}(OH)_r(A''H_p)_{3-nm+q}]_{1+p+q}$ Ia''

in which

A'' is pyridine which may be substituted by hydroxy, amino, chlorine, diethylamino, dimethylamino, hydroxyaminomethine, phenyl, pyrrolidino, piperidino or pyrrole-1-ylmethyl, preferably in the 4-position, pyrrole, imidazole, 1-methylimidazole, 4-methylimidazole, pyrazole, 4-methylpyrazole, triazole, 1-sodium pyrazole, 1-phenyltetrazole, 5-phenyltetrazole, adenine, guanine, purine or cytosine,

X'' is chlorine and

m, n, p, q and r are as defined in claim 1.

8. A process for the production according claim 5, 6 or 7, characterized in that a ruthenium(III) halide of the formula $RuX_3$, $RuX'_3$ or $RuX''_3$, where X, X' and X'' have the meanings defined in claims 5, 6 and 7 is reacted with the nitrogen heterocycle A, A' or A'' having the meanings defined in claims 5, 6 and 7.

9. A process as claimed in claim 8, characterized in that the reaction is carried out in a hydrohalic acid.

10. A process as claimed in claim 9, characterized in that the reaction is carried out in the mixture of a lower alkanol with aqueous hydrohalic acid.

11. Medicinal preparations containing one or more of the complex compounds claimed in any of claims 5 to 7.

12. sterile aqueous solutions containing one or more of the complex compounds claimed in any of claims 1 to 7.

13. The use of the compounds according to claims 1 to 7 for the production of medicinal preparations for the treatment of cancer.

14. A medicinal preparation comprising a pharmaceutically acceptable excipient and from 0.1 to 99.5 % by weight of at least one of the complex compounds according to claims 1 to 4.

**Revendications**

1. Composés de formule générale

$(BH_n)_m[RuX_{3+nm-q-r}(OH)_r(BH_p)_{3-nm+q}]_{1+a+q}$ I

dans laquelle
B est un hétérocycle basique, à un ou plusieurs noyaux, contenant un ou plusieurs atomes d'azote,
X est le chlore ou le brome,
m vaut 1 ou 2,
n vaut 1 ou 2, la somme de n et m n'étant pas supérieure à 3,
p vaut 0 ou 1, mais non 1 si n vaut 1,
q vaut 0 ou 1, mais non 1 si p vaut 1, et
r vaut 0 ou 1, pour utilisation dans un procédé thérapeutique.

2. Composés de formule générale I'

$(B'H_n)_m[RuX'_{3+nm-q-r}(OH)_r(B'H_p)_{3-nm+q}]_{1+a+q}$ I'

où
B' représente la pyridine, la pyrimidine, la pyridazine, la triazine, qui peuvent être substituées par un ou des radicaux hydroxy, amino, halogéno, alkyle en $C_1$-$C_4$, alcényle en $C_2$-$C_4$, (alkyle en $C_1$-$C_4$)-mercapto, formyle, (alcoxy en $C_1$-$C_4$)-carbonyle, (alcoxy en $C_1$-$C_4$)-carbonyl-(alkylène en $C_1$-$C_4$), di-(alkyle en $C_1$-$C_4$)-amino, di-(alkyle en $C_1$-$C_4$)-amino-(alkylène en $C_1$-$C_4$), di-(alkyle en $C_1$-$C_4$)-aminocarbonyle, di-(alkyle en $C_1$-$C_4$)-aminocarbonyl-(alkylène en $C_1$-$C_4$), hydroxyiminométhine, phényle, benzyle, benzoyle, pyrrolidino, pipéridino, pyrrol-1-yle ou pyrrol-1-yl-(alkylène en $C_1$-$C_4$) ou encore un cycle

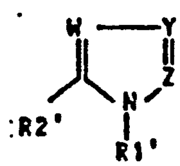

dans lequel
R1'est un hydrogène, un sodium un radical alkyle en $C_1$-$C_4$ ou le radical phényle,
R2' est un hydrogène ou un radical alkyle en $C_1$-$C_4$, amino, phényle, ou bien R1'et R2', pris ensemble, forment un groupe $(CH_2)_s$-, où s est un nombre entier de 4 à 8,
W est un azote ou CR3', où R3' est un hydrogène ou un radical alkyle en $C_1$-$C_4$, amino ou phényle, de préférence un hydrogène ou le radical méthyle,
Y est un azote ou CR4', où R4' est un hydrogène ou un radical alkyle en $C_1$-$C_4$, amino ou phényle, de préférence un hydrogène ou le radical méthyle,
Z est un azote ou CR5', où R5' est un hydrogène ou un radical alkyle en $C_1$-$C_4$, amino ou phényle, de préférence un hydrogène ou le radical méthyle, mais au moins l'un des radicaux R3', R4' ou R5' est un hydrogène,
la purine, l'adénine, la guanine ou la cytosine, et
X' est le chlore ou le brome, et
m, n, p, q et r ont les significations données dans la revendication 1, pour utilisation dans un procédé thérapeutique.

3. Composés de formule générale I''

$(B''H_n)_m[RuX''_{3+nm-q-r}(OH)_r(B''H_p)_{3-nm+q}]_{1+a+q}$ I''

où
B'' est la pyridine, qui peut être substituée, de préférence en position 4, par l'un des radicaux hydroxy, amino, chloro, diéthylamino, diméthylamino, hydroxyiminométhine, phényle, pyrrolidino, pipéridino ou pyrrol-1-ylméthyle; ou le pyrrole, l'imidazole, le 1-méthylimidazole, le 4-méthylimidazole, le pyrazole, le 4-méthylpyrazole, le triazole, le 1-sodiumpyrazole, le 1-phényltétrazole, le 5-phényltétrazole, l'adénine, la guanine, la purine ou la cytosine, et
X'' est le chlore, et
m, n, p, q et r ont les significations données dans la revendication 1, pour utilisation dans un procédé thérapeutique.

4. Imidazole pentachlororuthénate(III) de bisimidazolium, diimidazoletétrachlororuthénate(III) d'imidazolium, pyrazolehydroxotétrachlororuthénate(III) de bispyrazolium, (4-méthylpyrazole)hydroxotétrachlororuthénate(III) de bis-(4-méthylpyrazolium), pour utilisation dans un procédé thérapeutique.

5. Composés complexes de formule générale Ia

$(AH_n)_m[RuX_{3+nm-q-r}(OH)_r(AH_p)_{3-nm+q}]_{1+a+q}$ Ia

où
A est un hétérocycle basique, à un ou plusieurs noyaux, contenant un ou plusieurs atomes d'azote, à l'exception du pyrazole et de l'imidazole, et
X est le chlore ou le brome, et
m, n, p, q et r ont les significations données dans la revendication 1.

6. Composés complexes de formule générale Ia'

$(A'H_n)_m[RuX'_{3+nm-q-r}(OH)_r(A'H_p)_{3-nm+q}]_{1+a+q}$ Ia'

où
A' représente la pyridine, la pyrimidine, la pyridazine, la triazine, qui peuvent être substituées par un ou des radicaux hydroxy, amino, halogéno, alkyle en $C_1$-$C_4$, alcényle en $C_2$-$C_4$, (alkyle en $C_1$-$C_4$)-mercapto, formyle, (alcoxy en $C_1$-$C_4$)-carbonyle, (alcoxy en $C_1$-$C_4$)-carbonyl-(alkylène en $C_1$-$C_4$), di-(alkyle en $C_1$-$C_4$)-amino, di-(alkyle en $C_1$-$C_4$)-amino-(alkylène en $C_1$-$C_4$), di-(alkyle en $C_1$-$C_4$)-aminocarbonyle, di-(alkyle en $C_1$-$C_4$)-aminocarbonyl-(alkylène en $C_1$-$C_4$), hydroxyiminométhine, phényle, benzyle, benzoyle, pyrrolidino, pipéridino, pyrrol-1-yle ou pyrrol-1-yl-(alkylène en $C_1$-$C_4$), ou encore un cycle

dans lequel
R1'est un hydrogène un sodium, un radical alkyle en $C_1$-$C_4$ ou le radical phényle,
R2' est un hydrogène ou un radical alkyle en $C_1$-$C_4$, amino, phényle, ou bien R1'et R2', pris ensemble, forment un groupe $(CH_2)_s$, où s est un nombre entier de 4 à 8,
W est un azote ou CR3', où R3' est un hydrogène ou un radical alkyle en $C_1$-$C_4$, amino ou phényle, de préférence un hydrogène ou le radical méthyle,
Y est un azote ou CR4', où R4' est un hydrogène ou un radical alkyle en $C_1$-$C_4$, amino ou phényle, de préférence un hydrogène ou le radical méthyle,
Z est un azote ou CR5', où R5' est un hydrogène ou un radical alkyle en $C_1$-$C_4$, amino ou phényle, de préférence un hydrogène ou le radical méthyle, mais au moins l'un des radicaux R3', R4' ou R5' est un hydrogène, la purine, l'adénine, la guanine ou la cytosine, mais non pyrazole ou imidazole, et
X' est le chlore ou le brome, et
m, n, p, q et r ont les significations données dans la revendication 1.

7. Composés complexes de formule générale Ia''

$(A''H_n)_m[RuX''_{3+nm-q-r}(OH)_r(A''H_p)_{3-nm+q}]_{1+a+q}$ Ia''

où
A'' est la pyridine, qui peut être substituée, de préférence en position 4, par l'un des radicaux hydroxy, amino, chloro, diéthylamino, diméthylamino, hydroxyiminométhine, phényle, pyrrolidino, pipéridino ou pyrrol-1-ylméthyle; ou le pyrrole, le 1-méthylimidazole le 4-méthylimidazole, le 4-méthylpyrazole le triazole le sodiumpyrazole, le 1-phényltétrazole, le 5-phényltétrazole, l'adénine, la guanine, la purine ou la cytosine, et
X'' est le chlore, et
m, n, p, q et r ont les significations données dans la revendication 1.

8. Procédé de préparation selon les revendications 5, 6 ou 7, caractérisé en ce qu'on fait réagir un halogénure de ruthénium(III), respectivement de formules $RuX_3$, $RuX'_3$ et $RuX''_3$, où X, X' et X'' ont les significations données respectivement dans les revendications 5, 6 et 7 avec les hétérocycles azotés A, A' et A'' ayant les significations données respectivement dans les revendications 5, 6 et 7.

9. Procédé selon la revendication 8, caractérisé en ce qu'on réalise la réaction dans un milieu acidifié par un acide halogènhydrique.

10. Procédé selon la revendication 9, caractérisé en ce qu'on réalise la réaction dans un mélange d'un alcanol inférieur et d'un acide halogènhydrique en solution aqueuse.

11. Préparations pharmaceutiques contenant un ou plusieurs composés complexes selon l'une des revendications 5 à 7.

12. Solutions aqueuses stériles contenant un ou plusieurs composés complexes selon l'une des revendications 1 à 7.

13. Utilisation des composés indiqués dans les revendications 1 à 7 pour fabriquer des préparations pharmaceutiques destinées au traitement des maladies du type cancer.

14. Préparation pharmaceutique comportant un excipient pharmaceutiquement acceptable et de 0,1 à 99,5 % en poids d'au moins l'un des composés complexes mentionnés dans les revendications 1 à 4.